(19) Europäisches Patentamt
European Patent Office
Office européen des brevets

(11) Veröffentlichungsnummer: **0 153 640**
**A2**

# (12) EUROPÄISCHE PATENTANMELDUNG

(21) Anmeldenummer: 85101408.4

(22) Anmeldetag: 11.02.85

(51) Int. Cl.[4]: **C 07 C 39/17**

(30) Priorität: 22.02.84 DE 3406308

(43) Veröffentlichungstag der Anmeldung:
04.09.85 Patentblatt 85/36

(84) Benannte Vertragsstaaten:
CH DE FR GB IT LI SE

(71) Anmelder: BAYER AG
Konzernverwaltung RP Patentabteilung
D-5090 Leverkusen 1 Bayerwerk(DE)

(72) Erfinder: Blank, Heinz Ulrich, Dr.
Am Geusfelde 35
D-5068 Odenthal(DE)

(72) Erfinder: Silber, Gunter, Dr.
Gerstenkamp 21
D-5000 Köln 80(DE)

## (54) Verfahren zur Herstellung von 1.2.3.4.5.6.7.8-Octahydro-anthrahydrochinonen.

(57) Neues Verfahren zur Herstellung von 1.2.3.4.5.6.7.8-Octahydro-anthrahydrochinonen durch katalytische Hydrierung von Antrachinonen in Ethern.

EP 0 153 640 A2

Croydon Printing Company Ltd.

0153640

BAYER AKTIENGESELLSCHAFT 5090 Leverkusen, Bayerwerk
Konzernverwaltung RP
Patentabteilung B/Kü-c

# Verfahren zur Herstellung von 1.2.3.4.5.6.7.8-Octahydro-anthrahydrochinonen

Die Erfindung betrifft ein neues Verfahren zur Herstellung von 1.2.3.4.5.6.7.8-Octahydro-anthrahydrochinon durch katalytische Hydrierung von Antrachinon.

Verfahren zur Herstellung von 1.2.3.4.5.6.7.8-Octahydro-anthrahydrochinon durch katalytische Hydrierung von Anthrachinon sind bekannt. So ist in Chem. Ber. 58, 2685 (1925) ein Verfahren zur Herstellung von 1.2.3.4.5.6.7.8-Octahydro-anthrahydrochinon beschrieben, bei dem Anthrachinon in Gegenwart eines Platinkatalysators in Eisessig-Salzsäure bei 60°C unter einem Druck von 3 atm Wasserstoff hydriert wird. Das 1.2.3.4.5.6.7.8-Octahydro-anthrahydrochinon wird durch extraktive Aufarbeitung des Reaktionsgemisches isoliert. Bei der Nacharbeitung dieses Verfahrens wurde kein reines 1.2.3.4.5.6.7.8-Octahydroanthrahydrochinon, sondern nur ein Gemisch verschiedener Hydroanthrachinone erhalten und auch dieses nur in geringer Menge.

In Chem. Ber. 60, 2522 (1927) ist ein Verfahren zur Herstellung von 1.2.3.4.5.6.7.8-Octahydro-anthrahydrochinon beschrieben, bei dem Anthrachinon in Gegenwart von Raney-Nickel in Decalin bei 150°C und 150 bar Wasserstoffdruck hydriert wird. Für dieses Verfahren werden jedoch keine Ausbeuten angegeben. Außerdem wird in Anales Fis. y Chim. (Madrid) 40, 167 (1944) (CA 42, 7285 f) die katalytische Hydrierung von Anthrachinon mit Raney-Nickel in Methanol bei 200°C und 71 atm Wasserstoffdruck beschrieben. Bei dieser Hydrierung werden neben Octahydroanthrachinon auch 1.2.3.4.5.6.7.8-Octahydro-anthrahydrochinon isoliert. Eine Ausbeuteangabe fehlt jedoch.

Diese bekannten Verfahren haben den Nachteil, daß die Hydrierung des Anthrachinons nicht selektiv sondern uneinheitlich verläuft und daß infolgedessen das gewünschte Produkt, 1.2.3.4.5.6.7.8-Octahydro-anthrahydrochinon, nur in schlechten Ausbeuten entsteht und dies in Gemischen mit anderen Hydrierungsprodukten, aus denen sich das reine 1.2.3.4.5.6.7.8-Octahydro-anthrahydrochinon, wenn überhaupt, nur mit großem Aufwand isolieren läßt.

Es wurde nun gefunden, daß man 1.2.3.4.5.6.7.8-Octahydro-anthrahydrochinon in hoher Ausbeute und hoher Reinheit durch katalytische Hydrierung von Anthrachinon erhält, wenn man die Hydrierung des Anthrachinons in Ethern vornimmt. Überraschenderweise verläuft die Hydrierung des Anthrachinons in Ethern äußerst selektiv und liefert ein von anderen Hydrierungsprodukten

praktisch freies 1.2.3.4.5.6.7.8-Octahydro-anthrahydrochinon, das sich deshalb auch in einfacher Weise aus dem nach der Hydrierung anfallenden Reaktionsgemisch isolieren läßt. Mit dem gleichen Erfolg wie Anthrachinon lassen sich auch durch Alkylreste substituierte Anthrachinone in Ethern zu den entsprechenden durch Alkylreste substituierte Octahydro-anthrahydrochinonen hydrieren.

Die Erfindung betrifft daher ein Verfahren zur Herstellung von 1.2.3.4.5.6.7.8-Octahydro-anthrahydrochinonen der Formel

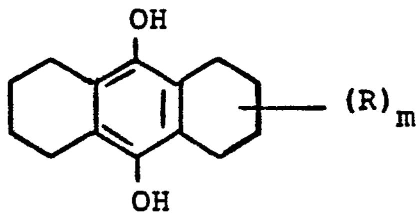

I

in der

R für einen Alkylrest. vorzugsweise einen $C_1$-$C_4$-Alkylrest, steht und

m 0, 1, 2 oder 3 bedeutet,

durch katalytische Hydrierung von Anthrachinonen der Formel

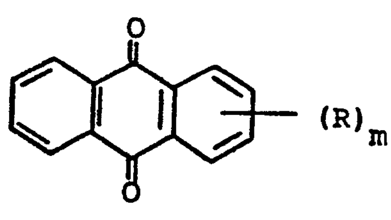

II

in der

R und m die unter Formel I angegebene Bedeutung haben,

in organischen Lösungsmitteln, das dadurch gekennzeichnet ist, daß man als Lösungsmittel Ether verwendet.

Als Ether kommen aliphatische und gemischt aliphatisch-aromatische Ether in Betracht.

Als aliphatische Ether seien beispielsweise genannt: offenkettige Ether wie Diethylether, Dipropylether, Diisopropylether, Dibutylether, Di-sekundär-butylether, tertiär Butyl-methylether, Diisopentyl-ether, Hexyl-ethyl-ether, Ethylenglykol-dimethylether, Ethylenglykol-methyl-tert.-butylether, Diethylenglykoldimethylether, Diethylenglykol-diethylether, Diethylenglykol-methyl-tert.-butyl-ether, Triethylenglykoldimethylether, Glycerin-1.2.3-trimethylether, Glycerin1.3-diethyl-2-methylether, Propylenglykol-dimethylether, Propylenglykoldiethylether, Dipropylenglykoldimethylether, Propylenglykol-methyl-tert.-butylether, 1,2-Butylenglykol-dimethylether, 1,4-Butandiol-dimethylether, 1,3-Butandiol-diethylether, 2,3-Butandiol-diethylether, Ethylenglykol-methylether-acetat, Ethylenglykol-ethylether-acetat, Dicyclohexylether, Methyl-cyclohexyl-ether, Ethyl-cyclohexyl-ether;
Cyclische Ether wie Dioxan und Tetrahydrofuran.

Als gemischt aliphatisch-aromatische Ether seien vor allem Anisol und Phenetol genannt.

Besonders bewährt haben sich die Glykolether der Formel

$$R_1O-[(CR_3R_4)_nO]_x-R_2 \qquad III$$

in der

$R_1$ für einen $C_1$-$C_4$-Alkyl- oder einen $C_1$-$C_5$-Alkanoyl-Rest,

$R_2$ für einen $C_1$-$C_4$-Alkyl-Rest und

$R_3$ und $R_4$ unabhängig voneinander für Wasserstoff oder einen $C_1$-$C_4$-Alkylrest, vorzugsweise einen $C_1$-$C_2$-Alkylrest, stehen und

n 2 oder 3 und

x 1 oder 2 bedeuten

und cyclische aliphatische Ether.

Bevorzugt werden Ethylenglykoldimethylether, Ethylenglykolmethyletheracetat und/oder Dioxan verwendet.

Die erfindungsgemäß zu verwendenden Ether werden in einer Menge von 2 bis 100, vorzugsweise 3 bis 30 Gew.-Teilen je Teil Anthrachinon eingesetzt. Die Menge Ether muß nicht unbedingt zum vollständigen Lösen

des Anthrachinons ausreichen; das Anthrachinon kann auch zum Teil suspendiert im Lösungsmittel vorliegen.

Für das erfindungsgemäße Verfahren eignen sich grundsätzlich alle bekannten Hydrierkatalysatoren, wie Raney-Nickel oder die Metalle der achten Nebengruppe des periodischen Systems wie Platin oder Palladium.

Die Metalle können gegebenenfalls auf geeignete anorganische Trägermaterialien, wie Kieselsäuren, Aluminiumoxiden, Aluminiumsilikaten, Calcium- oder Bariumcarbonaten oder Aktivkohle aufgebracht sein. Besonders bewährt hat sich für das erfindungsgemäße Verfahren jedoch die Verwendung von Raney-Nickel als Hydrierkatalysator. Die Verwendung von Raney-Nickel in Ethern verläuft besonders selektiv und führt zu einem besonders reinen Octahydro-anthrahydrochinon in besonders hohen Ausbeute.

Der Katalysator wird in üblichen Mengen von 0,1 bis 30 Gew.-% bezogen auf das hydrierende Anthrachinon eingesetzt. Bevorzugt werden 1 bis 10 Gew.-% Katalysator bezogen auf das zu hydrierende Anthrachinon verwendet.

Das erfindungsgemäße Verfahren wird bei Temperaturen von 20 bis 150°C, vorzugsweise bei Temperaturen von 40 bis 100°C durchgeführt. Zur Erzielung einer guten Selektivität ist das Arbeiten bei möglichst tiefen Temperaturen vorteilhaft.

Die erfindungsgemäße Hydrierung wird bei Wasserstoffdrucken von 1 bis 200 bar, vorzugsweise von 5 bis 150 bar durchgeführt. Besonders vorteilhafte Ergebnisse

erhält man, wenn man die Hydrierung bei 10 bis 150 bar Wasserstoffdruck durchführt.

Bei der erfindungsgemäßen Hydrierung werden Wasserstoffdruck und Temperatur vorzugsweise so aufeinander abgestimmt, daß die Hydrierung möglichst einheitlich, d.h. mit optimaler Selektivität verläuft. Arbeitet man bei niedrigeren Temperaturen, so kann ein höherer Wasserstoffdruck von Vorteil sein. Umgekehrt können niedrigere Wasserstoffdrucke eine erhöhte Temperatur erfordern.

Das erfindungsgemäße Verfahren kann diskontinuierlich oder kontinuierlich in bekannten Hydrierapparaturen, z.B. Hydrierautoklaven, Röhrenreaktoren, Umlaufanlagen oder Kesselkaskaden durchgeführt werden. Bei diskontinuierlicher Arbeitsweise legt man beispielsweise Anthrachinon, Lösungsmittel bzw. Lösungsmittelgemisch und Katalysator in einem Hydrierapparat vor und leitet dann bei der gewählten Reaktionstemperatur Wasserstoff unter erhöhtem Druck ein. Das Einleiten des Wasserstoffs kann dabei bis zum Erreichen des gewünschten Wasserstoffdruckes und/oder unter Aufrechterhaltung eines bestimmten Wasserstoffdruckes durch laufenden Ersatz des verbrauchten Wasserstoffs erfolgen. Die Hydrierung wird nach Aufnahme der berechneten Wasserstoffmenge abgebrochen. Bei kontinuierlicher Arbeitsweise wird Anthrachinon in dem ausgewählten Ether oder Ethergemisch gelöst bzw. suspendiert und die Lösung bzw. Suspension in einen Röhrenreaktor mit einem im Festbett- oder im Wirbelbett angeordneten Katalysator eindosiert und die Hydrierung in der Riesel- oder Sumpfphase durch Einleiten von Wasserstoff im Gleich- oder Gegenstrom durchgeführt.

Infolge der hohen Selektivität der erfindungsgemäßen Hydrierung gestaltet sich die Aufarbeitung des Hydriergemisches und die Isolierung des 1.2.3.4.5.6.7.8-Octahydro-anthrahydrochinons besonders einfach. Zur Isolierung der gewünschten Verbindung löst man das Hydrierungsprodukt durch Erwärmen des Hydrierungsgemisches - gegebenenfalls nach Zusatz von frischem Ether. Die Lösung wird in bekannter Weise, durch Filtrieren oder Zentrifugieren vom Katalysator befreit und das Lösungsmittel teilweise oder vollständig abdestilliert. Die sich nach dem Abkühlen des Sumpfes abscheidenden Kristalle werden in bekannter Weise isoliert. Das abdestillierte Lösungsmittel kann unmittelbar, das heißt ohne weitere Reinigung, für die nächste Hydrierung wiederverwendet werden.

Die auf diese Weise erhaltenen Kristalle können durch geringe Mengen an oxidativ gebildetem 1.2.3.4.5.6.7.8-Octahydro-anthrachinon leicht gelb verfärbt sein. Ein reines farbloses Produkt wird erhalten, indem man die abgetrennten Kristalle mit chlorierten Kohlenwasserstoffen, z.B. Methylenchlorid, 1,2-Dichlorethan, Chloroform oder Trichlorethylen wäscht. Im Hinblick auf die Oxidationsempfindlichkeit des Hydrochinons ist es vorteilhaft, bei der Aufarbeitung des Hydriergemisches sämtliche Operation in einer Inertgasatmosphäre (Stickstoffatmosphäre) durchzuführen.

Die 1.2.3.4.5.6.7.8-Octahydro-anthrahydrochinone sollen wie die 1.2.3.4-Tetrahydroanthrachinone als Antioxidantien für Kautschuk-Produkte verwendet werden.

Beispiel 1

In einem Hydrierautoklaven werden 68,64 g Anthrachinon in 400 ml Ethylenglykoldimethylether in Gegenwart von 5 g Raney-Nickel bei 80°C und 30 bar Wasserstoffdruck hydriert. Nach 3 Stunden ist die berechnete Menge Wasserstoff aufgenommen. Nach Abkühlen und Entspannen des Autoklaven wird das Hydriergemisch mit 400 ml Ethylenglykoldimethylether versetzt und unter Stickstoff auf Rückflußtemperatur erwärmt bis sämtliche organische Verbindungen in Lösung gegangen sind. Der Katalysator wird aus der Lösung durch Absaugen der Suspension über eine Fritte abgetrennt. Nach dem Auswaschen des abfiltrierten Katalysators mit 200 ml Ethylenglykoldimethylether werden aus den vereinigten Filtraten insgesamt 700 ml Ethylenglykoldimethylether unter Stickstoff abdestilliert. Der Destillationssumpf wird anschließend auf 0 bis 5°C abgekühlt. Das auskristallisierte Produkt wird abgesaugt, mit etwa 100 ml Methylenchlorid gewaschen und getrocknet.

Es werden 56,57 g 1.2.3.4.5.6.7.8-Octahydro-anthrahydrochinon in Form farbloser Nadeln vom Fp.: 239-241°C erhalten. Beim Aufarbeiten der Mutterlauge werden noch weitere 3,2 g 1.2.3.4.5.6.7.8-Octahydro-anthrahydrochinon in Form eines farblosen Kristall-Mehls vom Fp.: 238 - 240°C gewonnen. Gesamtausbeute an 1.2.3.4.5.6.7.8-Octahydro-anthrahydrochinon: 83 % der Theorie.

Bei Verwendung von Ethylenglykoldiethylether als Lösungsmittel wurde ein vergleichbares Ergebnis erhalten.

Bei Verwendung von Dioxan als Lösungsmittel wurden 56,01 g 1.2.3.4.5.6.7.8-Octahydro-anthrahydrochinon in Form farbloser Kristalle vom Fp.: 239-241°C erhalten. Aus der Mutterlauge ließen sich weitere 1,82 g der Verbindung vom Fp.: 238-240°C gewinnen. Ein vergleichbares Resultat wurde bei der Hydrierung in Tetrahydrofuran erhalten.

Wurde die Hydrierung bei

a) 40°C und 100 bar Wasserstoffdruck
b) 40°C und 150 bar Wasserstoffdruck
c) 100°C und 10 bar Wasserstoffdruck

vorgenommen, so betrugen die Zeiten bis zur Aufnahme der berechneten Menge Wasserstoff

a) 4,5 h
b) 3,5 h
c) 4 h

und die Ausbeuten an 1.2.3.4.5.6.7.8-Octahydro-anthrahydrochinon

a) 67,45 g (96 %-iges Produkt) = 90 % der Theorie
b) 66,2 g (96 %-iges Produkt) = 88 % der Theorie
c) 49,1 g (100 %-iges Produkt) = 68 % der Theorie.

Beispiel 2

In einem Hydrierautoklaven werden 68,64 g Anthrachinon in 400 ml Ethylenglykol-methylether-acetat in Gegenwart von 5 g Raney-Nickel bei 80°C und 30 bar Wasserstoffdrucks hydriert. Nach 5 Stunden ist die berechnete Menge Wasserstoff aufgenommen. Nach Abkühlen und Entspannen des Autoklaven wird das Hydriergemisch mit 100 ml Dioxan aus dem Autoklaven gespült.
Die Suspension wird nach Zusatz weiterer 100 ml Dioxan solange auf 110°C erwärmt, bis sich die ausgefallenen organischen Verbindungen vollständig gelöst haben. Der Katalysator wird durch Absaugen der Suspension über eine Fritte abgetrennt und mit 200 ml Dioxan gewaschen. Aus den vereinigten Filtraten werden insgesamt 650 ml Lösungsmittelgemisch unter Stickstoff abdestilliert. Der Destillationssumpf wird anschließend auf 0°C abgekühlt und mit 100 ml Methylenchlorid verrührt. Dann wird das kristallisierte Produkt abgesaugt, mit Methylenchlorid gewaschen und getrocknet. Es werden 65,03 g 1.2.3.4.5.6.7.8-Octahydro-anthrahydrochinon in Form farbloser Kristalle vom Fp.: 238-240°C erhalten. Das Produkt ist 98 %ig. Ausbeute an 1.2.3.4.5.6.7.8-Octahydro-anthrahydrochinon: 88 % der Theorie.

Wurde anstelle von Ethylenglykol-methylether-acetat Phenetol als Lösungsmittel verwendet, so wurden 53,9 g 1.2.3.4.5.6.7.8-Octahydro-anthrahydrochinon in Form farbloser Kristalle vom Fp.: 222-225°C erhalten. Gemäß NMR ist das Produkt 90 %ig, es enthält noch 10 % Tetrahydroanthrachinon.

Wurde anstelle von Ethylenglykol-methylether-acetat die gleiche Menge an Diisopropylether oder Dibutylether verwendet, so wurden 50,16 g 92 %iges 1.2.3.4.5.6.7.8-Octahydro-anthrahydrochinon in Form farbloser Kristalle gewonnen. Das Produkt enthält noch 8 % Tetrahydroanthrachinon.

Beispiel 3

In einem Hydrierautoklaven werden 73,33 g 2-Methylanthrachinon in 350 ml Ethylenglykoldimethylether in Gegenwart von 5 g Raney-Nickel bei 80°C und 30 bar Wasserstoffdruck hydriert. Nach 4 Stunden ist die berechnete Wasserstoffmenge aufgenommen. Man läßt abkühlen, entspannt den Autoklaven und nimmt das Hydriergut in 420 ml Dioxan auf. Die Suspension wird unter Stickstoff auf Rückflußtemperatur erhitzt, bis sämtliche organischen Verbindungen in Lösung gegangen sind. Der Katalysator wird aus der heißen Lösung abfiltriert und mit 430 ml Dioxan gewaschen. Aus dem Filtrat werden 1030 ml Lösungsmittelgemisch abdestilliert. Der Destillationsrückstand wird abgekühlt und mit 200 ml n-Hexan verrührt. Das ausgefallene Produkt wird abgesaugt und anschließend getrocknet. Es werden 64,2 g (= 84 % der Theorie) 2-Methyl-1.2.3.4.5.6.7.8-octahydro-anthrahydrochinon in Form eines farblosen Kristall-Mehls vom Fp 191-193°C erhalten.

Beispiel 4

Es wird wie in Beispiel 3 beschrieben gearbeitet, nur werden statt der 73,33 g 2-Methyl-anthrachinon 77,97 g 2-Ethyl-anthrachinon verwendet. Es werden 66,34 g (= 82 % der Theorie) 2-Ethyl-1.2.3.4.5.6.7.8-octahydro-anthrahydrochinon in Form eines leicht gelblich gefärbten Kristall-Mehls vom Fp 171-174°C erhalten.

Patentansprüche

1. Verfahren zur Herstellung von 1.2.3.4.5.6.7.8-Octahydro-anthrahydrochinonen der Formel

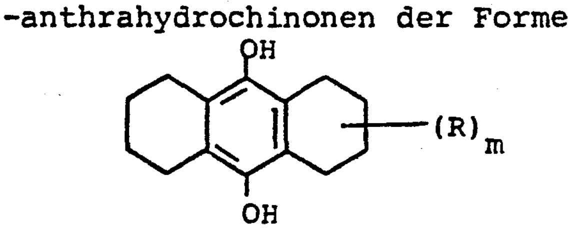

I

in der

R für einen Alkylrest steht und

m 0, 1, 2 oder 3 bedeutet,

durch katalytische Hydrierung von Anthrachinonen der Formel

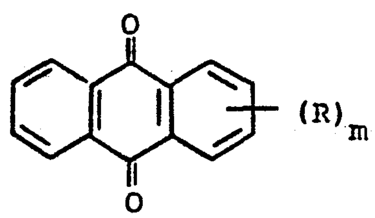

II

in der

R und m die unter Formel I angegebene Bedeutung haben

in organischen Lösungsmitteln, dadurch gekennzeichnet, daß man als Lösungsmittel Ether verwendet.

2. Verfahren gemäß Anspruch 1, dadurch gekennzeichnet, daß man aliphatische und/oder gemischt-aliphatisch-aromatische Ether verwendet.

3. Verfahren gemäß Anspruch 1, dadurch gekennzeichnet, daß man als Ether Glykolether der Formel

$$R_1O-[(CR_3R_4-)_nO]_x \quad R_2 \qquad III$$

in der

$R_1$ für einen $C_1$-$C_4$-Alkyl- oder einen $C_1$-$C_5$-Alkanoyl-Rest,

$R_2$ für einen $C_1$-$C_4$-Alkylrest und

$R_3$ und $R_4$ unabhängig voneinander für Wasserstoff oder einen $C_1$-$C_4$-Alkylrest stehen und

n 2 oder 3 und

x 1 oder 2 bedeuten,

oder cyclische aliphatische Ether verwendet.

4. Verfahren gemäß Anspruch 1, dadurch gekennzeichnet, daß man als Ether Ethylenglykoldimethylether, Ethylenglykolmethyletheracetat und/oder Dioxan verwendet.

5. Verfahren gemäß Ansprüchen 1 bis 4, dadurch gekennzeichnet, daß man als Hydrierkatalysator Raney-Nickel verwendet.

6. Verfahren gemäß Ansprüchen 1 bis 5, dadurch gekennzeichnet, daß man die Hydrierung bei Temperaturen von 20-150°C durchführt.

7. Verfahren nach Ansprüchen 1 bis 6, dadurch gekennzeichnet, daß man die Hydrierung bei Drucken von 5-150 bar vornimmt.